# EUROPEAN PATENT SPECIFICATION

(11) **EP 3 106 872 B1**
(45) Date of publication and mention of the grant of the patent: **09.08.2017**
(21) Application number: 15172431.7
(22) Date of filing: 16.06.2015
(51) Int. Cl.: G01N 33/497, B60K 28/06

(54) **A BREATH ANALYSER DEVICE AND A RELATED METHOD**
ATEMANALYSEVORRICHTUNG UND ZUGEHÖRIGES VERFAHREN
DISPOSITIF ANALYSEUR D'HALEINE ET PROCÉDÉ ASSOCIÉ

(43) Date of publication of application: 21.12.2016
(73) Proprietor: Autoliv Development AB, 447 83 Vårgårda (SE)
(72) Inventor: Hök, Bertil, 723 44 Västerås (SE); Gester, Raimo, 723 35 Västerås (SE)
(74) Representative: Parry, Simon James

(56) References cited:
- JP-A- 2008 191 871
- JP-A- 2009 090 691
- JP-A- 2011 220 759
- US-A1- 2008 170 762

## Description

The present invention relates to a breath analyser device, and more particularly relates to a tamper evident breath analyser device. The invention also relates to a tamper-evident method of analysing a breath sample.

It is important in a number of situations to be able accurately to analyse the exhaled breath of a test subject. The most common reason for doing this is to detect the presence of alcohol in the test subject's breath, which will of course be indicative of the test subject having a raised blood-alcohol level and thus impaired judgement and reaction times. Testing for alcohol in this way is very important for safety reasons in a large number of situations including, for example, the operation of heavy or dangerous machinery, the operation of aircraft, and the operation of motor vehicles. Breath testing for alcohol is now widely used in the area of law enforcement and is routinely carried out on motor vehicle drivers on the road networks of most major countries, with strict penalties in place for drivers found to be driving under the influence of alcohol.

Alcohol is a major factor in a very large number of road accidents, and so great efforts are made to reduce the incidence of driving under the influence of alcohol. One proposal which is now being considered more widely is the provision of so-called "alco-locks" in motor vehicles which will prevent operation of the motor vehicle until an approved breath sample having an alcohol content below a predetermined maximum threshold value has been given by the driver. It is envisaged that the device of the present invention will be particularly useful in such alco-lock arrangements. However, it is to be appreciated that the invention is not limited to use in alco-lock arrangements, and could find wider application in the field of alcohol breath testing. The present invention could be more widely employed in breath analysis more generally, and may not even be restricted to testing for the presence of alcohol. Nevertheless, the present invention is described herein with specific reference to alcohol testing.

Whilst alcohol testing for evidentiary of diagnostic purposes is generally achieved by infrared spectroscopy which is very accurate, a simpler method of alcohol testing is normally used for screening purposes; for example at the roadside by law enforcement personnel, or in the case of alco-locks installed in vehicles. Alcohol testing for screening purposes has therefore previously been achieved via catalysis; for example using fuel cells or semiconductor devices. These types of device are advantageous in terms of production cost, but have been found to suffer from problems of unreliability. The catalytic function is difficult to control, and the sensors have a limited lifetime. Furthermore, devices of this type require a test subject to deliver forced expiration into a tight-fitting mouthpiece which can be problematic for people with impaired respiratory function, and will generally be inconvenient and off-putting for drivers in the case of vehicle alco-locks.

It has therefore been proposed to provide improved breath analyser devices that do not require physical contact between the device and the test subject. This type of device is particularly advantageous in the case of alco-locks because it can be conveniently positioned on the dashboard, steering wheel, or A-post of a motor vehicle for receipt of a breath sample from a driver test subject in a normal, or reasonably normal, driving position. However, testing in this manner will of course mean that the breath sample received by the device will be diluted with ambient air.

US2013/0231871 A1 proposes a type of no-contact breath analyser device which address the issue of ambient air dilution of the breath sample by measuring the concentration of a tracer substance such as carbon dioxide within the sample in order to estimate the degree of dilution and thereby allow the estimation of the true breath concentration of alcohol.

Breath analyser devices of the type described above which do not require physical contact between the device and the test subject can be particularly susceptible to tampering. This can be a particular problem in the case of devices which are configured as alco-locks in motor vehicles. For example, it has been found that test subjects have been known to attempt to "cheat" such devices by interfering with their breath samples upstream of the device so as to cause the device to provide a false negative alcohol signal. One known tamper strategy involves blowing a breath sample along a length of cooled tubing between the test subject's mouth and the sample inlet of the breath analyser device so that the test subject's breath sample will be cooled as it passes along the length of tubing before entering the device. Another known strategy involves blowing a breath sample through a length of tubing filled with charcoal in a similar manner. Both of these strategies involve delaying alcohol-polluted breath from entering the device until after the device has given a negative signal on the basis of unpolluted air initially present in the tube. It is therefore desirable to provide a breath analyser device which is able to detect these types of tamper strategies and thereby avoid providing a false negative alcohol signal in such situations.

JP2008191871 A proposes a breath analyser device which is configured to detect attempts to provide spurious breath samples.

JP2011220759A proposes a breath analyser device which is configured to monitor the test subject's lung movement.

It is an object of the present invention to provide an improved breath analyser device. It is another object of the present invention to provide an improved method of analysing a breath sample.

According to a first aspect of the present invention, there is provided a tamper evident breath analyser device having an inlet region and a sensor arrangement, the inlet region being configured to receive a breath sample from a test subject via an inlet port and direct the sample to the sensor arrangement, the sensor arrangement being configured to provide a signal representative of the concentration of a volatile substance within said sample, the device including an imaging device arranged and configured to capture images of a test subject's face when the device is in use and positioned to receive a breath sample from the test subject via said inlet port, the device further comprising a processor unit operable to analyse said images and produce a signal indicative of an untampered breath sample in dependence on said analysis, wherein said processor unit is configured to: i) identify a test subject's mouth and to determine whether or not the mouth is open by analysing the length of an outline contour of the subject's mouth in said images; and ii) detect the occurrence of a blocking object between a test subject's mouth and the breath analyser device by detecting a break in said outline contour of the subject's mouth in said images, and to produce an error signal in response thereto.

Conveniently, said imaging device is located adjacent said inlet port.

The imaging device may comprise a CCD image sensor or a CMOS image sensor.

Preferably, said processor unit is configured to analyse said images by comparing individual said images to one another.

Advantageously, said processor unit is configured to run a facial identification algorithm to identify facial features in said images.

The breath analyser device may comprise a data buffer configured to receive and temporarily store said images for subsequent analysis by the processor unit.

Advantageously, the sensor arrangement includes a carbon dioxide sensor arranged and configured to provide a carbon dioxide signal representative of the concentration of carbon dioxide in an air flow produced by said breath sample and directed through the sensor arrangement; and said processor unit is operable, substantially concurrently with analysis of said images; to compare said breath sample to a predetermined threshold value.

Conveniently, said sensor arrangement includes a carbon dioxide sensor arranged and configured to provide a carbon dioxide signal representative of the concentration of carbon dioxide in an air flow produced by said breath sample and directed through the sensor arrangement; said processor unit being operable to compare said carbon dioxide signal to a predetermined threshold value, and to retrieve said images from the data buffer and perform said analysis on the retrieved images in response to said carbon dioxide signal exceeding said threshold value.

Optionally, said processor unit is operable to determine a point in time at which said carbon dioxide signal exceeds said threshold value, and to retrieve from said data buffer and analyse a series of said images corresponding to a predetermined time period at least the beginning of which precedes said point in time.

According to another aspect of the present invention, there is provided a tamper-evident method of analysing a breath sample, the method comprising the steps of: providing a breath analyser device having an inlet region and a sensor arrangement, the inlet region being configured to receive a breath sample from a test subject via an inlet port and direct the sample to the sensor arrangement, the sensor arrangement being configured to provide a signal representative of the concentration of a volatile substance within the sample; the method further involving the provision of an imaging device directed towards the face of a test subject, operating said imaging device whilst the test subject provides a breath sample through said inlet port so as to capture images of the test subject's face, and analysing said images to produce a signal indicative of an untampered breath sample in dependence on said analysis, the method being characterised by: i) involving the identification of a test subject's mouth and determining whether or not the mouth is open by analysing the length of an outline contour of the subject's mouth in said images, and ii) involving the detection of a blocking object between a test subject's mouth and said breath analyser device by detecting a break in said outline contour of the subject's mouth in said images, and producing an error signal in response thereto.

Preferably, the method further involves analysing said images by comparing individual images to one another.

Advantageously, the method further involves identifying facial features in said images.

Advantageously, the method further involves the step of temporarily storing said images from the imaging device in a data buffer prior to said step of analysing the images.

Optionally, the method involves, substantially concurrently with said step of analysing the images: using a carbon dioxide sensor to provide a carbon dioxide signal representative of the concentration of carbon dioxide in said air flow, and comparing said carbon dioxide signal to a predetermined threshold value
Alternatively, the method may further comprise the steps of: using a carbon dioxide sensor to provide a carbon dioxide signal representative of the concentration of carbon dioxide in said air flow; comparing said carbon dioxide signal to a predetermined threshold value; and, if said carbon dioxide signal exceeds said predetermined threshold value, retrieving said stored images from the data buffer and performing said analysis on the retrieved images. The method may further comprise the steps of: determining a point in time at which said carbon dioxide signal exceeds said threshold value; and retrieving from said data buffer and analysing a series of images corresponding to a predetermined time period, at least the beginning of which precedes said point in time.

Said predetermined time period may be at least 2 seconds.

Said predetermined time period may begin at least 1 second before said point in time.

So that the invention may be more readily understood, and so that further features thereof may be appreciated, embodiments of the invention will now be described by way of example with reference to the accompanying drawings in which:
Figure 1 is a perspective view showing an exemplary breath analyser device;
Figure 2 is a schematic illustration showing the device positioned for use relative to a test subject;
Figure 3 is a schematic illustration showing internal componentry of the device;
Figure 4 comprises two graphs showing exemplary signal patterns relating to carbon dioxide and alcohol levels during use of the device;
Figure 5 shows in more detail an exemplary arrangement of components within part of the device;
Figure 6 is a schematic front elevational view of a test subject, as viewed from the device in the position illustrated in figure 2, showing the test subject's mouth closed;
Figure 7 is a view similar to that of figure 6, but which shows the test subject's mouth open to exhale a breath sample;
Figure 8 is a view similar to that of figure 7, but which shows a disturbing object indicative of tampering partially obstructing the test subject's open mouth;
Figure 9 is a schematic flow chart showing a possible operational regime of the device in accordance with the present invention;
Figure 10 is another schematic flow chart, similar to the one of figure 9, but which shows an alternative possible operational regime; and
Figure 11 shows a series of graphs displaying respective data plotted against time and which pertain to operation of the device.

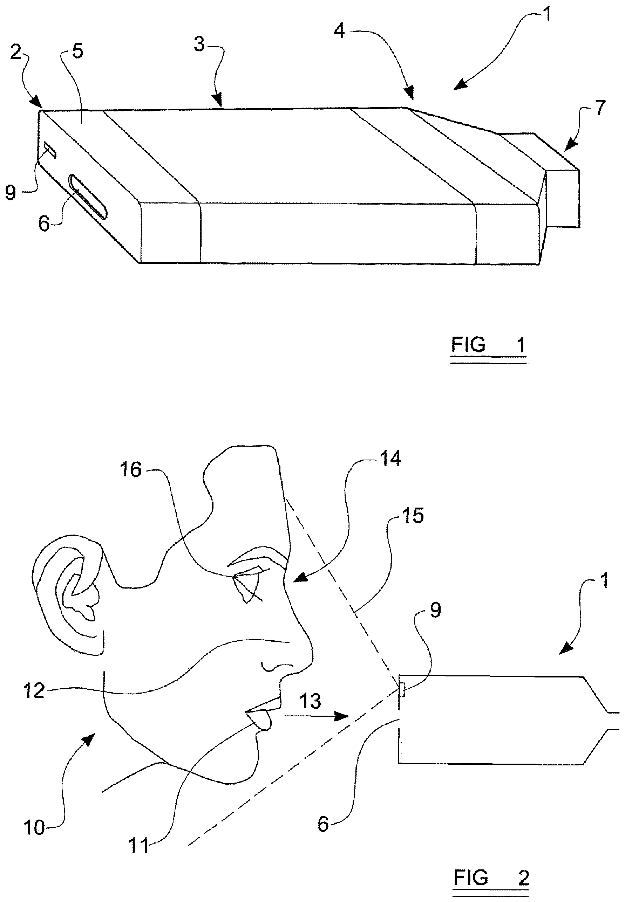

Turning now to consider the drawings in more detail, figure 1 illustrates the general arrangement of a breath analyser device 1 in accordance with an embodiment of the present invention. In general terms, the device comprises an inlet region 2, a sensor module 3 which incorporates a sensor arrangement, and an outlet region 4. The device 1 may be configured to operate in accordance with the principles disclosed in US2013/0231871A1, such that the sensor arrangement of the sensor module 3 may be configured in a similar manner to the sensor arrangement of the device proposed therein, as will be described in more detail below. However, other configurations of sensor arrangement are also possible. In all embodiments it is envisaged that the sensor arrangement will be configured to provide a signal representative of the concentration of at least one volatile substance within a breath sample.

The inlet region 2 comprises an inlet housing 5 having a sample inlet port 6 arranged at one end of the device 1. The inlet port 6 is open to atmospheric air outside the device 1 and is configured to receive a breath sample from a test subject. As will be explained in more detail below, the inlet region 2 of the device is configured to direct the breath sample to the sensor arrangement within the sensor module 3 of the device 1 for analysis.

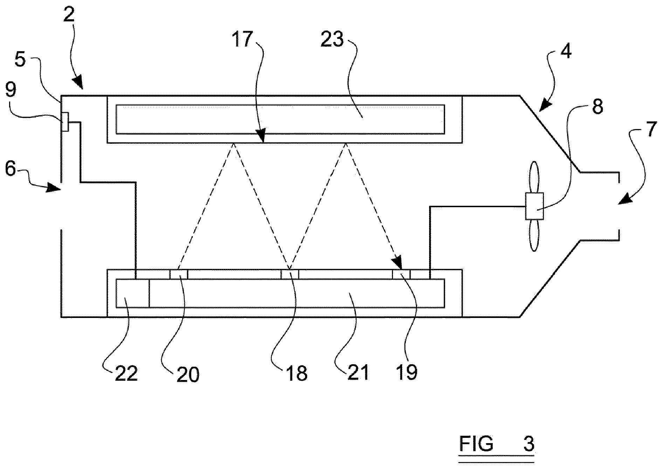

The outlet region 4 of the device has an exhaust port (indicated generally at 7, but not visible in figure 1) at the opposite end of the device 1 to the sample inlet port 6, through which the diluted breath sample is exhausted to the atmosphere after passing through, and being analysed within, the sensor module 3. The outlet region 4 may be provided with an internal battery-operated fan 8 (not shown in figure 1, but illustrated schematically in figure 3) to pull the gas through the device 1.

The inlet region 2 of the device is provided with a small (typically less than 5 mm across in its longest dimension) imaging device 9 which may be a digital device such as a charge-coupled device (CCD) image sensor or a complementary metal-oxide-semiconductor (CMOS) image sensor. The imaging device 9 may either be simply mounted externally on the housing 5 of the inlet region 2, or it may alternatively be accommodated in a recess formed in the housing 5 so as to be substantially flush with an outer surface of the inlet region 2 as illustrated in figure 2. As will be noted, the imaging device 9 is positioned so as to face outwardly from the end surface of the inlet region 2 within which the sample inlet port 6 is formed, and in the arrangement illustrated is located adjacent the inlet port 6. The imaging device 9 may be provided in combination with a lens and other standard optical elements to define an appropriate field of view, as will be described in more detail below.

Figure 2 illustrates a typical operational position of the breath analyser device 1 relative to a test subject 10 from whom a breath sample is to be provided to the device 1. As will be noted, the device is positioned in spaced relation to the text subject's mouth 11 such that a breath sample exhaled from the test subject's mouth 11 and/or nose 12 (typically at a velocity of 0.5 to 2 m/s) will be directed generally towards the sample inlet port 6 as indicated by airflow arrow 13. It is envisaged that in normal use the device 1 will be positioned or held approximately 10 to 15 cm from the test subject's face 14.

Figure 2 also shows, in dashed lines, the field of view 15 of the imaging device 9, as defined by its optical arrangement. As illustrated, the imaging device 9 is thus positioned such that when the device 1 is arranged in an appropriate position relative to the test subject 10 to receive a breath sample therefrom via the inlet port 6, the major parts of the test subject's face 14, such as his or her mouth 11, nose 12, and eyes 16 will fall within the imaging device's field of view 15. In the particular arrangement illustrated, it will be noted that the imaging device 9 is positioned above the sample inlet port 6 when the device 1 is orientated in the in-use position shown. This is considered advantageous because of course it means that the test subject's mouth 11, which is towards the bottom of his or her face 14, may be generally aligned with the sample inlet port 6 of the device whilst the major proportion of the test subject's face 14 (most of which is located above the mouth 11) is positioned within a field of view 15 of modest angle. However, it is envisaged that in other embodiments the imaging device 9 could be located to one side of the inlet port 6, or even below the inlet port 6, whilst still encompassing most of the test subject's face 14 within its field of view 15. Such an arrangement may, however, require an imaging device 9 with a somewhat wider field of view 15 than that illustrated in figure 2 in order to ensure capture of the main parts of the test subject's face 14.

Figure 3 illustrates an exemplary layout of internal componentry of the device 1, although it is to be noted that the layout illustrated is in no way limiting and it is envisaged that alternative arrangements may be used instead. Nevertheless, the arrangement of figure 3 is useful in understanding the manner in which embodiments of the device operate.

As will be noted, the sensor module 3 comprises an internal chamber 17 which fluidly interconnects the inlet and outlet regions 2, 4 of the device 1 and thus defines a flow passage through the device along which a breath sample will be directed in use of the device.

In the illustrated embodiment of the device 1 there is provided a pair of sensors 18, 19 inside the sensor module 3 and to one side of the internal chamber 17 therethrough. One of the sensors 19 is responsive to the volatile substance of interest (for example ethanol in the case of an alcohol breath analyser), and the other sensor 18 is responsive to CO₂ which will be used as a tracer substance according to the principles taught previously in US2013/0231871.

In the proposed embodiment, the sensor module 3 also includes a source 20 of electromagnetic radiation within the infrared (IR) wavelength range and so the sensors 18, 19 are provided in the form of IR detectors equipped with band pass type interference filters tuned to the wavelength intervals which coincide with absorption peaks of the substances which each respective sensor is configured to sense. For ethanol and CO₂ suitable wavelength intervals are 9.5 ± 0.3 µm and 4.26 ± 0.05 µm respectively.

As illustrated schematically by the dashed arrow in figure 3, the sensors 18, 19 receive an IR beam emitted by the source 20 after reflections against the inner wall of the internal chamber 17, which is preferably covered by a thin film of gold or aluminium or another highly reflecting material. The IR beam is reflected once before reaching the CO₂ sensor 18, and is reflected three times before reaching the ethanol detector 19. Thus the optical path is much longer for the ethanol sensor 19 than for the CO₂ sensor 18, resulting in a higher sensitivity to absorption.

As also illustrated schematically in figure 3, the IR source 20, the two sensors 18, 19 and the imaging device 9 are all electrically and operatively connected to a processor unit 21, with the imaging device 9 being connected thereto via a memory buffer 22. The processor unit 21 preferably includes integrated analogue and digital circuit elements for signal processing and control. Preferably one or several microprocessors are included for signal processing and management of signals to a display (not shown) for indication of measurement results.

The data buffer 22 is configured to temporarily store image signals produced by the imaging device 9. As will be described in more detail hereinafter, the imaging device 9 and the processor unit 21 are used to derive an indication as to whether or not a breath sample directed into the inlet port 6 of the device has been tampered with, for example by the provision of a tamper device (such as a length of tubing) between the test subject's mouth 11 and the inlet port 6 of the device. However, it is first relevant to consider the basic function of the device in measuring breath alcohol levels.

The breath analyser device 1 may be provided in the form of an autonomous handheld unit, in which case power is provided by a battery 23 as illustrated schematically in figure 2. However in other embodiments, the device 1 can be provided within the instrument panel of a motor vehicle, or within the interior trim of a motor vehicle generally in the region of the driver's normal sitting position, and may be operable in combination with other equipment of the vehicle. In such an arrangement it is envisaged that the device may be embodied in a so-called alco-lock arrangement. In either case it is envisaged that the device will not require physical contact with the test subject in order to function. As already indicated, and illustrated in figure 2, it is envisaged that the device may operate at a range of approximately 10 to 15 cm from the test subject's face 14. As explained above, this means that a breath sample directed into the device from the test subject's mouth 11 will be diluted with ambient air.

Estimation of the dilution of the breath sample is performed via the use of CO₂ as a tracer substance (although it is to be appreciated that it is within the scope of the present invention to use other tracer substances such as, for example, water vapour). The partial pressure of CO₂ within deep (alveolar) breath air is typically 4.8 kPa, corresponding to 4.8% by volume, whereas the background ambient concentration seldom exceeds 0.1 % v/v. The degree of dilution therefore can be calculated from the ratio CO₂ₐₗᵥ / CO₂ₘₑₐₛ, where CO₂ₐₗᵥ and CO₂ₘₑₐₛ represent the alveolar and measured concentrations, respectively. The variability of CO₂ₐₗᵥ between different individuals expressed as one standard deviation is relatively modest; typically of the order of 10% of the average.

Proposed devices in accordance with embodiments of the present invention function by multiplying the measured concentration of a substance in a diluted breath sample by CO₂ₐₗᵥ / CO₂ₘₑₐₛ, in order to obtain an estimated value of the undiluted concentration. This mode of operation is extremely rapid and convenient for the test subject.

Transfer from a screening mode of operation into a mode of higher measuring accuracy may be accomplished by identifying an undiluted breath using the CO₂ sensor 18. In the absence of a signal indicating an undiluted sample (which would be the case, for example, if the test subject 10 were to place his or her mouth 11 directly over the inlet port 6 or to fit a length of tube or a mouthpiece to the inlet port 6 and blow directly into it), an estimation of the sample dilution is used in the calculation of the substance concentration.

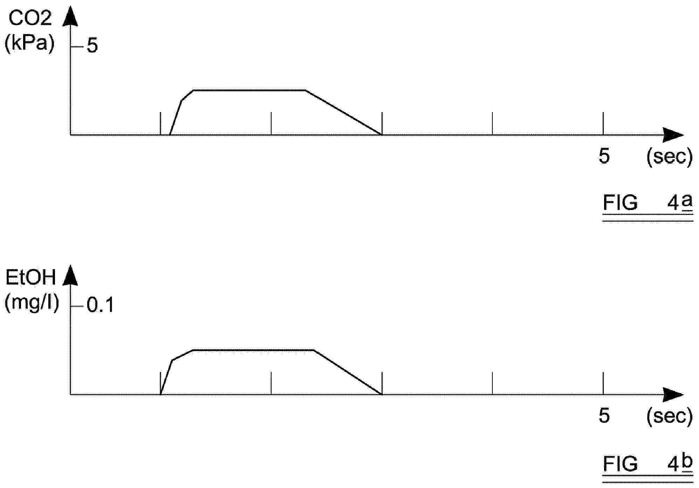

Figure 4 schematically shows the signal patterns when performing breath tests in which the test subject 10 does not have his or her mouth 11 in direct fluid connection with the inlet port 6 of the device; i.e. when the test subject's mouth 11 is spaced from the inlet port 6 as illustrated in figure 2. More particularly, figure 4(a) graphically shows the variation of the measured concentration of CO₂ as a function of time, whilst figure 4(b) graphically shows the variation of the substance of primary interest, in this case ethanol (EtOH), as a function of time during a breath test. Both signals are basically zero at the start of the test, and grow to a maximum during the expiratory phase, before then returning to zero as the sensing unit is ventilated. When the CO₂ concentration reaches its maximum, the processor unit's algorithm will assume a dilution ratio of CO₂ₐₗᵥ / CO₂ₘₑₐₛ and multiply it with the measured ethanol concentration at that time in order to obtain the estimated undiluted EtOH concentration. The entire course of the test shown graphically in Figure 4 has a duration of only a few seconds, which is due to the fact that the test subject 10 is instructed to terminate the expiration when the processor 21 determines that a certain threshold value V of CO₂ has been reached; which may be for example 2 kPa, corresponding to a dilution ratio of approximately 2.4. The CO₂ and EtOH signals must coincide in time, as shown, in order to be attributed to the breath of a test subject.

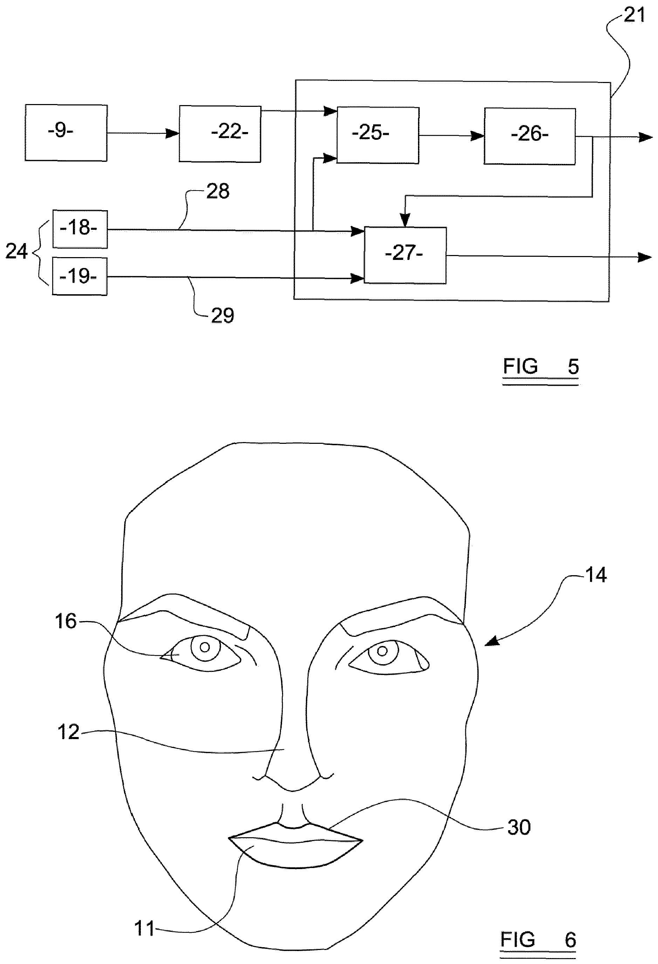

Turning now to consider figure 5, there is shown a more detailed schematic illustration of the processor unit 21 and its connections to the data buffer 22 (and via which also to the image sensor 9), the CO₂ sensor 18, and the ethanol sensor 19. Collectively, the CO₂ sensor 18 and the ethanol sensor 19 may be considered to represent a breath sensor 24. The processor unit 21 illustrated in figure 5 also includes an image processor 25, an image classifier 26 and a breath signal processor 27 (which is configured to function and perform the analysis described above), each of which may be provided in the form of respective microprocessors. It is to be noted, however, than in other embodiments it is envisaged that the image classifier 26 could be integrated with the image processor 25.

As illustrated, the CO₂ sensor 18 and the ethanol sensor 19 will produce respective signals 28, 29, which are both fed to the breath signal processor 27 for analysis in the manner described above. Additionally, the CO₂ signal 28 may also be fed to the image processor 25 as shown. As will be appreciated, the image processor 25 is configured to process image data, which may be retrieved from the data buffer 22.

It is envisaged that the imaging device 9 will produce real time image signals with a relatively large bandwidth, including typically more than 100 x 100 pixels at a frame rate of 10 images per second. When activated, the imaging device 9 will capture a plurality of successive images of the test subject's face 14 for temporary storage in the data buffer 22. The image processor 25 may then retrieve the captured images from the data buffer 22 for subsequent comparative analysis. The image processor 25 may thus be provided in the form of a general purpose microprocessor with the capacity to perform algorithms at relatively fast speed. The image processor 25 operates in concert with the image classifier 26, whose function is to classify a breath sample from a test subject 10 as either approved or disapproved on the basis of decision criteria related to the comparative analysis of the captured images of the test subject's face.

Figure 6 is a schematic representation of an image of the test subject's face 14, as might be captured by the imaging device 9. As will be apparent, the principal features of the test subject's face 14 such as mouth 11, nose 12, and eyes 16, are all clearly visible. The image processor 25 runs a facial identification algorithm which is effective to identify these, and possibly other, facial features in the captured images.

As will also be noted, the test subject's mouth 11 is shown in the image depicted in figure 6 to be closed. The test subject's mouth may be identified by the image processor 25 in response to the detection of a closed line 30 of distinct contrast in the image, said line defining the outline of the mouth 11 against the surrounding skin tones of the test subject's face 14.

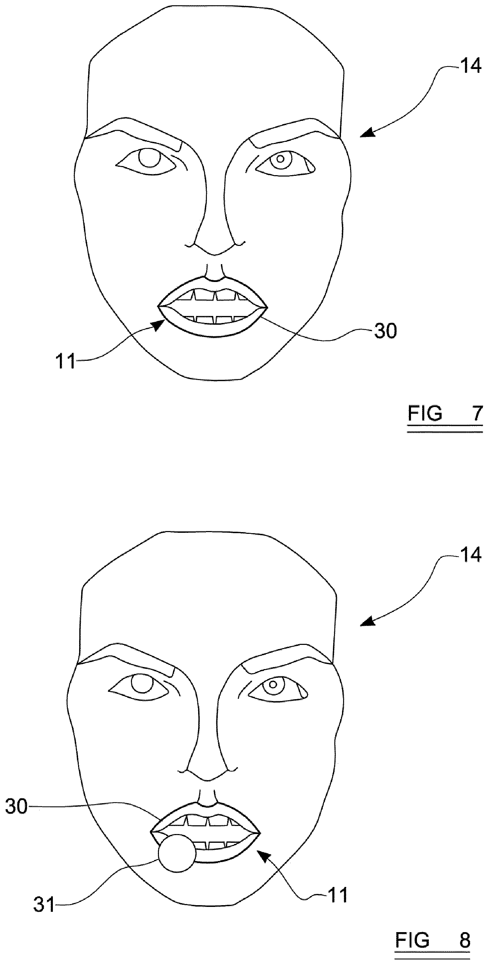

Figure 7 illustrates an exemplary subsequent image of the test subject's face 14, as captured by the imaging device 9. As will be immediately apparent, in this image the test-subject's mouth 11 is open, as will occur for example when the test subject 10 opens his or her mouth in response to a prompting signal (for example visual or audible) from the device 1 in readiness to exhale a breath sample. The image processor 25 is configured, via its facial identification algorithm, to identify that the test subject's mouth 11 has been opened, by comparing the image with preceding images (such as that depicted in figure 6) and identifying a lengthening of the closed line 30 of distinct contrast which represents the outline of the mouth 11.

As will be appreciated, to provide a genuine and untampered breath sample, the test subject 10 is required to exhale a breath through his or her open mouth towards the inlet aperture 6 of the breath analyser device 1. In doing this, there should be no reason for the person to place any external object between his or her mouth 14 and the analyser device 1. However, as already indicated above, some known techniques to attempt to interfere with proper analysis of breath involve blowing through a length of tubing or the like, which would thus require the test subject 10 to position the tubing between his or her mouth 14 and the analyser device 1. The device of the present invention, and more particularly the image processor 25 and the image classifier 26, are configured to detect the occurrence of any such external object in the facial images captured by the imaging device 9.

Figure 8 is a schematic representation of an image of a test subject's face 14 which may be captured by the imaging device 9 as the test subject 10 brings an external object such as length of tubing 31 up towards his or her mouth 14 in readiness to attempt to interfere with the proper functioning of the analyser device 1 by blowing a breath sample through the tubing. An image of this nature, which will be indicative to the image classifier 26 of a tamper attempt, is characterised by the normally closed line 30 of contrast which denotes the outline of the mouth 14 becoming broken by the external object 31 (such as a tube). The external object 31 can thus be considered to represent a blocking object in the sense that it blocks the normally closed line 30 denoting the outline of the test subject's mouth. The algorithm which is run by the image processor 25 is thus configured to detect any such break in the closed line 30, which will thus result in the image classifier classifying the resulting breath sample as one which has been tampered with and, as such, should be rejected.

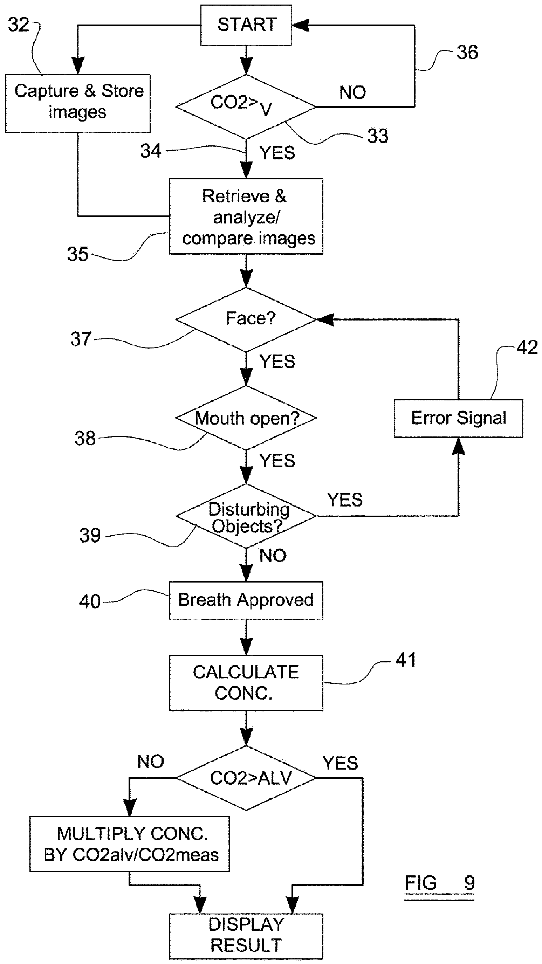

Figure 9 shows an outline flow diagram representative of a possible operating regime of the analyser device 1. Initially, a test subject 10 will be prompted (for example via an audible or visual signal produced by the device 1) to position his or her face 14 in front of the device 1 as illustrated in figure 2, whereupon the imaging device 9 will begin to capture a plurality of images of the subject's face 14 for temporary storage in the data buffer 22, as denoted at 32. The test subject 10 will also, either at the same time or after a slight delay, be prompted to direct an exhaled breath sample into the inlet port 6, during which time the imaging device 9 will continue to capture successive images for storage in the data buffer 22. At the same time, the breath signal processor 27 will function to estimate the dilution of the breath sample, as denoted at 33. If the breath signal processor 27 determines that a CO₂ signal 28 received from the CO₂ sensor 18 is representative of the CO₂ concentration in the sample exceeding the predetermined threshold value V (e.g. 2% v/v), then the image processor 25 will be prompted (as denoted at 34) to retrieve and analyse the images which have been captured and stored in the data buffer 22 (as denoted at 35). If, on the other hand, the breath signal processor 27 determines that the CO₂ signal received from the CO₂ sensor 18 is not representative of the CO₂ concentration in the sample exceeding the predetermined threshold value V, then it will return an error signal, as denoted at 36.

Following determination by the breath signal processor 27 that the CO₂ signal received from the CO₂ sensor 18 is representative of the CO₂ concentration in the sample exceeding the predetermined threshold value V, the image processor 25 will proceed to analyse the images by comparing successive images of the captured series to one another, to thereby systematically identify whether or not a face 14 is present in the imaging sensor's field of view 15 (as denoted at 37), whether or not the subject's mouth 11 is open (as denoted at 38), and whether or not a blocking or disturbing object such as a tube 31 is present between the subject's mouth 11 and the breath analyser device (as denoted at 39), as described above. If no such blocking or disturbing object is identified then the breath sample will be approved (as denoted at 40) and the breath signal processor will be instructed (as denoted at 41) to proceed to calculate the concentration of volatile substance (e.g. ethanol) in the breath sample as previously described. However, if a blocking or disturbing object 31 is detected, then the image processor 25 will return an error signal 42, which may be shown on a display, and will be tasked with identifying a test subject's face 14 again for a subsequent test attempt.

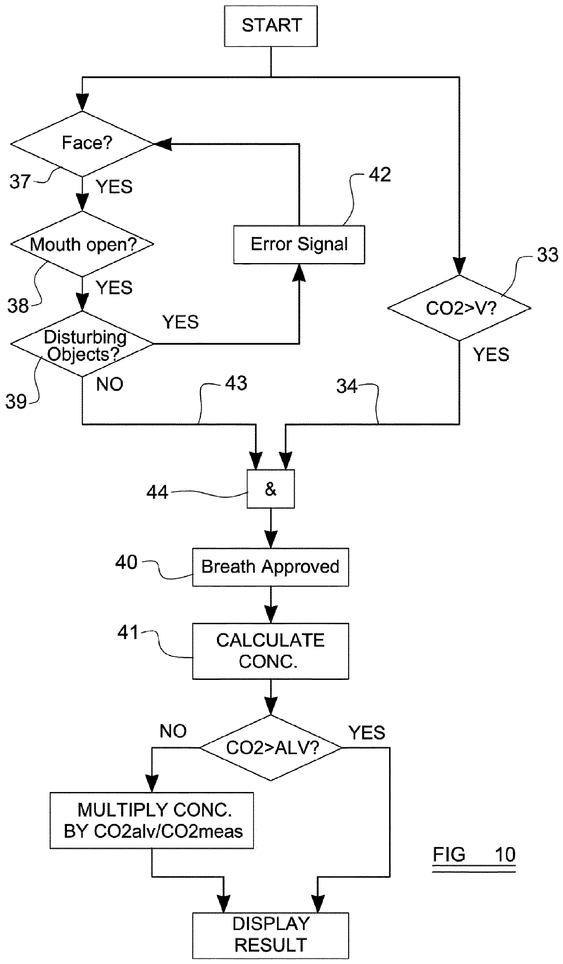

Turning now to consider figure 10, there is shown an outline flow diagram representative of another possible operating regime of the analyser device 1. In this proposal, the test subject 10 will be prompted (for example via an audible or visual signal produced by the device 1) to position his or her face 14 in front of the device 1, whereupon the imaging device 9 will begin to capture a plurality of images of the subject's face 14 for temporary storage in the data buffer. The test subject 10 will also, either at the same time or after a slight delay, be prompted to direct an exhaled breath sample into the inlet port 6, during which time the imaging device 9 will continue to capture successive images for storage in the data buffer 22. During this process of image capture, the image processor 25 will begin and proceed to analyse the images by comparing successive images of the captured series to one another, to thereby systematically identify whether or not a face 14 is present in the imaging sensor's field of view 15 (as denoted at 37), whether or not the subject's mouth 11 is open (as denoted at 38), and whether or not a blocking or disturbing object such as a tube 31 is present between the subject's mouth 11 and the breath analyser device (as denoted at 39), as described above. If no such blocking or disturbing object is identified then the image processor 25 will pass an "image-approved" signal 43 to an AND gate, as illustrated at 44 in figure 10. However, if a blocking or disturbing object 31 is detected, then the image processor 25 will return an error signal 42, which may be shown on a display, and will be tasked with identifying a test subject's face 14 again for a subsequent test attempt.

Substantially concurrently with the above-described image analysis, the breath signal processor 27 will function to estimate the dilution of the breath sample, as denoted at 33. If the breath signal processor 27 determines that a CO₂ signal 28 received from the CO₂ sensor 18 is representative of the CO₂ concentration in the sample exceeding the predetermined threshold value V (e.g. 2% v/v), then it will pass a "CO₂ concentration approved" signal 34 to the above-mentioned AND gate 44 The system will approve a breath sample (as denoted at 40) upon the production of both an "image-approved" signal 43 and a "CO₂ concentration approved" signal 34, whereupon the breath signal processor 27 will be instructed (as denoted at 41) to proceed to continue the concentration of volatile substance (e.g. ethanol) in the breath sample as previously described. If, on the other hand, the breath signal processor 27 determines that the CO₂ signal 28 received from the CO₂ sensor 18 is not representative of the CO₂ concentration in the sample exceeding the predetermined threshold value V, then it will return an error signal.

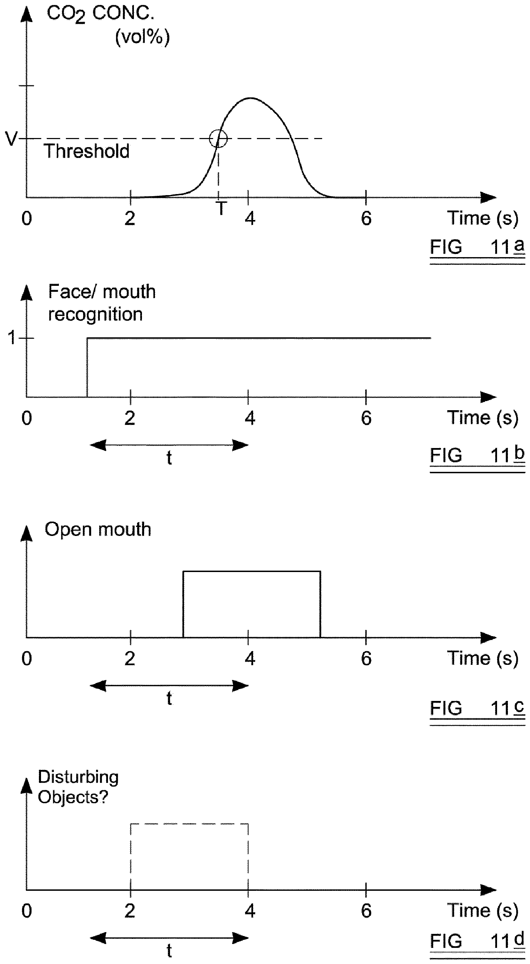

Turning now to consider figure 11, there are shown a series of graphs relating to a typical breath sample test conducted via the method proposed above. All of the graphs display time in seconds along their x-axes. Figure 11 (a) shows the variation of the measured concentration of CO₂ as a function of time, and is thus similar to figure 4(a). As will be observed, the CO₂ concentration begins to peak at a time of approximately 3 seconds, and exceeds the predetermined threshold value V at a time T of approximately 3.6 seconds.

Figure 11 (b) denotes detection by the image processor 25 of the face 14 and/or mouth 11 of the test subject 10. As will therefore be noted, the signal displayed in the y-axis goes high (i.e. from 0 to 1 as denoted) when the face and/or mouth is detected, which in this illustrated example occurs at approximately 1 second. As will be appreciated, this precedes the CO₂ concentration exceeding the predetermined threshold value V because the test subject's face 14 must enter the imaging device's field of view 15 before the breath sample is received by the breath analyser device. In the illustrated example, it will be noted that the test subject's face/mouth is detected approximately 2.6 seconds before the CO₂ concentration exceeds the predetermined threshold value V.

Figure 11 (c) denotes detection by the image processor 25 of the test subject's mouth 14 opening prior to exhalation of a breath sample, which will of course also occur before the CO₂ concentration exceeds the predetermined threshold value V. In this example, the test subject's mouth 14 is detected as opening at approximately 3 seconds.

Figure 11 (d) denotes detection by the image processor 25 of the test subject's mouth 14 becoming obscured by a blocking or disturbing object such as a length of tubing 31 or the like. In the example illustrated, the blocking object is detected at a time of 2 seconds, which again precedes the point in time at which the CO₂ concentration exceeds the predetermined threshold value V. In the case of the type of operating regime described above with reference to figure 9, in which the image processor 25 is prompted to retrieve and analyse the images which have been captured and stored in the data buffer 22 only in response to the breath signal processor 27 determining that the CO₂ signal received from the CO₂ sensor 18 is representative of the CO₂ concentration in the sample exceeding the predetermined threshold value V, it is therefore proposed that the breath signal processor 27 will be operable to determine the point in time, denoted T on the graph of figure 11 (a) at which the CO₂ concentration exceeds the predetermined threshold value V, such that the image processor 25 will, in response thereto, then retrieve from the data buffer 22 a series of images captured by the imaging device 9 which correspond to a predetermined time period (an example of which is denoted t in figures 11 (b), (c), (d)), at least the beginning of which precedes said point in time T. This is effective to ensure that the image processor 25 maybe tasked with analysing the correct series of images, in which it is most likely that a blocking object 31 will be detected in the event of a tampering attempt. In some embodiments it is proposed that this predetermined time period may be at least 2 seconds in duration, and may begin at least 1 second before the point in time T at which the CO₂ concentration is deemed to exceed the predetermined threshold value V.

When used in this specification and claims, the terms "comprises" and "comprising" and variations thereof mean that the specified features, steps or integers are included. The terms are not to be interpreted to exclude the presence of other features, steps or integers.

While the invention has been described in conjunction with the exemplary embodiments described above, many modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention as defined by the appended claims.

## Claims

1. A tamper evident breath analyser device (1) having an inlet region (2) and a sensor arrangement, the inlet region (2) being configured to receive a breath sample from a test subject (10) via an inlet port (6) and direct the sample to the sensor arrangement, the sensor arrangement being configured to provide a signal representative of the concentration of a volatile substance within said sample, the device (1) including an imaging device (9) arranged and configured to capture images of a test subject's face (14) when the device (1) is in use and positioned to receive a breath sample from the test subject (10) via said inlet port (6), the device (1) further comprising a processor unit (21) operable to analyse said images and produce a signal indicative of an untampered breath sample in dependence on said analysis, the device being **characterised in that** said processor unit (21) is configured to: i) identify a test subject's mouth (11) and to determine whether or not the mouth (11) is open by analysing the length of an outline contour (30) of the subject's mouth (11) in said images; and ii) detect the occurrence of a blocking object (31) between a test subject's mouth (11) and the breath analyser device (1) by detecting a break in said outline contour (30) of the subject's mouth in said images, and to produce an error signal in response thereto.

2. A device according to claim 1, wherein said processor unit (21) is configured to analyse said images by comparing individual said images to one another.

3. A device according to any preceding claim, wherein said processor unit (21) is configured to run a facial identification algorithm to identify facial features (11, 12, 16) in said images.

4. A device according to any preceding claim, further comprising a data buffer (22) configured to receive and temporarily store said images for subsequent analysis by the processor unit (21).

5. A device according to any preceding claim, wherein the sensor arrangement includes a carbon dioxide sensor (18) arranged and configured to provide a carbon dioxide signal (28) representative of the concentration of carbon dioxide in an air flow produced by said breath sample and directed through the sensor arrangement; and said processor (21) unit is operable, substantially concurrently with analysis of said images; to compare said breath sample to a predetermined threshold value (V).

6. A device according to claim 4, wherein said sensor arrangement includes a carbon dioxide sensor (18) arranged and configured to provide a carbon dioxide signal (28) representative of the concentration of carbon dioxide in an air flow produced by said breath sample and directed through the sensor arrangement; said processor unit (21) being operable to compare said carbon dioxide signal (28) to a predetermined threshold value (V), and to retrieve said images from the data buffer (22) and perform said analysis on the retrieved images in response to said carbon dioxide signal (28) exceeding said threshold value (V).

7. A device according to claim 6, wherein said processor unit (21) is operable to determine a point in time (T) at which said carbon dioxide signal (28) exceeds said threshold value (V), and to retrieve from said data buffer (22) and analyse a series of said images corresponding to a predetermined time period (t) at least the beginning of which precedes said point in time (T).

8. A tamper-evident method of analysing a breath sample, the method comprising the steps of: providing a breath analyser device (1) having an inlet region (2) and a sensor arrangement, the inlet region (2) being configured to receive a breath sample from a test subject via an inlet port (6) and direct the sample to the sensor arrangement, the sensor arrangement being configured to provide a signal representative of the concentration of a volatile substance within the sample; the method further involving the provision of an imaging device (9) directed towards the face (14) of a test subject (10), operating said imaging device (9) whilst the test subject (10) provides a breath sample through said inlet port (6) so as to capture images of the test subject's face (14), and analysing said images to produce a signal indicative of an untampered breath sample in dependence on said analysis, the method being **characterised by**: i) involving the identification of a test subject's mouth (11) and determining whether or not the mouth (11) is open by analysing the length of an outline contour (30) of the subject's mouth in said images; and ii) involving the detection of a blocking object (31) between a test subject's mouth (11) and said breath analyser device (1) by detecting a break in said outline contour (30) of the subject's mouth in said images, and producing an error signal in response thereto.

9. A method according to claim 8, further involving analysing said images by comparing individual images to one another.

10. A method according to claim 8 or claim 9, further involving identifying facial features (11, 12, 16) in said images.

11. A method according to any one of claims 8 to 10, further involving the step of temporarily storing said images from the imaging device in a data buffer (22) prior to said step of analysing the images.

12. A method according to any one of claims 8 to 11, wherein the method involves, substantially concurrently with said step of analysing the images: using a carbon dioxide sensor (18) to provide a carbon dioxide signal (28) representative of the concentration of carbon dioxide in said air flow, and comparing said carbon dioxide signal (28) to a predetermined threshold value (V).

13. A method according to claim 11, the method further comprising the steps of: using a carbon dioxide sensor (18) to provide a carbon dioxide signal (28) representative of the concentration of carbon dioxide in said air flow; comparing said carbon dioxide signal (28) to a predetermined threshold value (V); and, if said carbon dioxide signal (28) exceeds said predetermined threshold value, retrieving said stored images from the data buffer (22) and performing said analysis on the retrieved images.

14. A method according to claim 13, further comprising the steps of: determining a point in time (T) at which said carbon dioxide signal (28) exceeds said threshold value (V); and retrieving from said data buffer (22) and analysing a series of images corresponding to a predetermined time period (t), at least the beginning of which precedes said point in time (T).

## Patentansprüche

1. Manipulationssichere Atemanalysevorrichtung (1) mit einem Einlassbereich (2) und einer Sensoranordnung, wobei der Einlassbereich (2) so ausgelegt ist, dass er eine Atemprobe von einer Testperson (10) über eine Einlassöffnung (6) erhält und die Probe zur Sensoranordnung leitet, wobei die Sensoranordnung so ausgelegt ist, dass sie ein Signal liefert, das die Konzentration einer flüchtigen Substanz in der Probe darstellt, wobei die Vorrichtung (1) eine Abbildungsvorrichtung (9) aufweist, die so angeordnet und ausgelegt ist, dass sie Bilder vom Gesicht (14) einer Testperson aufnimmt, wenn die Vorrichtung (1) verwendet wird, und so platziert ist, dass sie eine Atemprobe von der Testperson (10) über die Einlassöffnung (6) erhält, wobei die Vorrichtung (1) ferner eine Prozessoreinheit (21) umfasst, die so einsetzbar ist, dass sie die Bilder analysiert und in Abhängigkeit von der Analyse ein Signal erzeugt, dass eine nicht manipulierte Atemprobe anzeigt, wobei die Vorrichtung **dadurch gekennzeichnet ist, dass** die Prozessoreinheit (21) so ausgelegt ist, dass sie:
i) den Mund (11) einer Testperson erkennt und feststellt, ob der Mund (11) offen oder nicht offen ist, indem sie die Länge eines Außenumrisses (30) des Munds (11) der Person in den Bildern analysiert;
und ii) das Vorhandensein eines blockierenden Gegenstands (31) zwischen dem Mund (11) einer Testperson und der Atemanalysevorrichtung (1) erkennt, indem sie eine Unterbrechung in dem Außenumriss (30) des Munds der Person in den Bildern erkennt, und als Reaktion darauf ein Fehlersignal erzeugt.

2. Vorrichtung nach Anspruch 1, wobei die Prozessoreinheit (21) so ausgelegt ist, dass sie die Bilder durch Vergleichen einzelner Bilder miteinander analysiert.

3. Vorrichtung nach einem der vorhergehenden Ansprüche, wobei die Prozessoreinheit (21) so ausgelegt ist, dass sie zum Erkennen von Gesichtsmerkmalen (11, 12, 16) in den Bildern einen Gesichtserkennungsalgorithmus anwendet.

4. Vorrichtung nach einem der vorhergehenden Ansprüche, die ferner einen Datenpuffer (22) umfasst, der so ausgelegt ist, dass er die Bilder für eine spätere Analyse durch die Prozessoreinheit (21) empfängt und vorübergehend speichert.

5. Vorrichtung nach einem der vorhergehenden Ansprüche, wobei die Sensoranordnung einen Kohlenstoffdioxidsensor (18) aufweist, der so angeordnet und ausgelegt ist, dass er ein Kohlenstoffdioxidsignal (28) liefert, das die Konzentration von Kohlenstoffdioxid in einem Luftstrom darstellt, der durch die Atemprobe erzeugt wird und durch die Sensoranordnung geleitet wird; und wobei die Prozessoreinheit (21) so einsetzbar ist, dass sie im Wesentlichen gleichzeitig mit einer Analyse der Bilder die Atemprobe mit einem vorgegebenen Schwellenwert (V) vergleicht.

6. Vorrichtung nach Anspruch 4, wobei die Sensoranordnung einen Kohlenstoffdioxidsensor (18) aufweist, der so angeordnet und ausgelegt ist, dass er ein Kohlenstoffdioxidsignal (28) liefert, das die Konzentration von Kohlenstoffdioxid in einem Luftstrom darstellt, der durch die Atemprobe erzeugt wird und durch die Sensoranordnung geleitet wird; wobei die Prozessoreinheit (21) so einsetzbar ist, dass sie das Kohlenstoffdioxidsignal (28) mit einem vorgegebenen Schwellenwert (V) vergleicht und die Bilder aus dem Datenpuffer (22) abruft und die Analyse an den abgerufenen Bildern als Reaktion darauf vornimmt, dass das Kohlenstoffdioxidsignal (28) den Schwellenwert (V) überschreitet.

7. Vorrichtung nach Anspruch 6, wobei die Prozessoreinheit (21) so einsetzbar ist, dass sie einen Zeitpunkt (T) bestimmt, zu dem das Kohlenstoffdioxidsignal (28) den Schwellenwert (V) überschreitet, und aus dem Datenpuffer (22) eine Reihe der Bilder, die einem vorgegebenen Zeitraum (t) entsprechen, abruft und analysiert, wobei zumindest dessen Anfang vor dem Zeitpunkt (T) liegt.

8. Manipulationssicheres Verfahren zum Analysieren einer Atemprobe, wobei das Verfahren folgende Schritte umfasst: Bereitstellen einer Atemanalysevorrichtung (1) mit einem Einlassbereich (2) und einer Sensoranordnung, wobei der Einlassbereich (2) so ausgelegt ist, dass er eine Atemprobe von einer Testperson über eine Einlassöffnung (6) erhält und die Probe zur Sensoranordnung leitet, wobei die Sensoranordnung so ausgelegt ist, dass sie ein Signal liefert, das die Konzentration einer flüchtigen Substanz in der Probe darstellt; wobei das Verfahren ferner das Bereitstellen einer Abbildungsvorrichtung (9), die auf das Gesicht (14) einer Testperson (10) gerichtet ist, ein Betätigen der Abbildungsvorrichtung (9), während die Testperson (10) durch die Einlassöffnung (6) eine Atemprobe liefert, damit sie Bilder des Gesichts (14) der Testperson aufnimmt, und ein Analysieren der Bilder zum Erzeugen eines Signals in Abhängigkeit von der Analyse umfasst, das eine nicht manipulierte Atemprobe anzeigt, wobei das Verfahren **dadurch gekennzeichnet ist, dass** es: i) ein Erkennen des Munds (11) einer Testperson und ein Feststellen umfasst, ob der Mund (11) offen oder nicht offen ist, indem die Länge eines Außenumrisses (30) des Munds der Person in den Bildern analysiert wird; und ii) ein Erkennen eines blockierenden Gegenstands (31) zwischen dem Mund (11) einer Testperson und der Atemanalysevorrichtung (1) durch Erkennen einer Unterbrechung in dem Außenumriss (30) des Munds der Person in den Bildern und ein Erzeugen eines Fehlersignals als Reaktion darauf umfasst.

9. Verfahren nach Anspruch 8, das ferner ein Analysieren der Bilder durch Vergleichen einzelner Bilder miteinander umfasst.

10. Verfahren nach Anspruch 8 oder Anspruch 9, das ferner ein Erkennen von Gesichtsmerkmalen (11, 12, 16) in den Bildern umfasst.

11. Verfahren nach einem der Ansprüche 8 bis 10, das ferner den Schritt des vorübergehenden Speicherns der Bilder aus der Abbildungsvorrichtung in einem Datenpuffer (22) vor dem Schritt des Analysierens der Bilder umfasst.

12. Verfahren nach einem der Ansprüche 8 bis 11, wobei das Verfahren, im Wesentlichen gleichzeitig mit dem Schritt des Analysierens der Bilder Folgendes umfasst: Verwenden eines Kohlenstoffdioxidsensors (18) zum Liefern eines Kohlenstoffdioxidsignals (28), das die Konzentration von Kohlenstoffdioxid in dem Luftstrom darstellt, und Vergleichen des Kohlenstoffdioxidsignals (28) mit einem vorgegebenen Schwellenwert (V).

13. Verfahren nach Anspruch 11, wobei das Verfahren ferner folgende Schritte umfasst: Verwenden eines Kohlenstoffdioxidsensors (18) zum Liefern eines Kohlenstoffdioxidsignals (28), das die Konzentration von Kohlenstoffdioxid in dem Luftstrom darstellt; Vergleichen des Kohlenstoffdioxidsignals (28) mit einem vorgegebenen Schwellenwert (V); und, wenn das Kohlenstoffdioxidsignal (28) den vorgegebenen Schwellenwert überschreitet, Abrufen der gespeicherten Bilder aus dem Datenpuffer (22) und Durchführen der Analyse an den abgerufenen Bildern.

14. Verfahren nach Anspruch 13, das ferner folgende Schritte umfasst: Bestimmen eines Zeitpunkts (T), zu dem das Kohlenstoffdioxidsignal (28) den Schwellenwert (V) überschreitet; und Abrufen aus dem Datenpuffer (22) und Analysieren einer Reihe von Bildern, die einem vorgegebenen Zeitraum (t) entsprechen, wobei zumindest dessen Anfang vor dem Zeitpunkt (T) liegt.

## Revendications

1. Dispositif analyseur d'haleine (1) inviolable comportant une zone d'entrée (2) et un ensemble capteur, la zone d'entrée (2) étant configurée pour recevoir un échantillon d'haleine d'un sujet testé (10) par le biais d'un orifice d'entrée (6) et diriger l'échantillon vers l'ensemble capteur, l'ensemble capteur étant configuré pour fournir un signal représentant la concentration d'une substance volatile dans ledit échantillon, le dispositif (1) comprenant un dispositif d'imagerie (9) agencé et configuré pour capturer des images d'un visage (14) du sujet testé quand le dispositif (1) est utilisé et positionné pour recevoir un échantillon d'haleine du sujet testé (10) par le biais dudit orifice d'entrée (6), le dispositif (1) comprenant en outre une unité centrale (21) pouvant être mise en oeuvre pour analyser lesdites images et produire un signal indiquant un échantillon d'haleine non falsifié en fonction de ladite analyse, le dispositif étant **caractérisé en ce que** ladite unité centrale (21) est configurée pour : i) identifier une bouche (11) du sujet testé et déterminer si la bouche (11) est ouverte ou non en analysant la longueur d'un contour extérieur (30) de la bouche (11) du sujet dans lesdites images ; et ii) détecter la présence d'un objet de blocage (31) entre une bouche (11) du sujet testé et le dispositif analyseur d'haleine (1) en détectant une rupture dans ledit contour extérieur (30) de la bouche du sujet dans lesdites images, et produire un signal d'erreur en réaction à cela.

2. Dispositif selon la revendication 1, dans lequel ladite unité centrale (21) est configurée pour analyser lesdites images en comparant des images individuelles entre elles.

3. Dispositif selon l'une quelconque des revendications précédentes, dans lequel ladite unité centrale (21) est configurée pour exécuter un algorithme de reconnaissance faciale afin de reconnaître des traits de visage (11, 12, 16) dans lesdites images.

4. Dispositif selon l'une quelconque des revendications précédentes, comprenant en outre une mémoire tampon (22) configurée pour recevoir et enregistrer temporairement lesdites images pour une analyse subséquente par l'unité centrale (21).

5. Dispositif selon l'une quelconque des revendications précédentes, dans lequel l'ensemble capteur comprend un capteur de dioxyde de carbone (18) agencé et configuré pour fournir un signal relatif au dioxyde de carbone (28) représentant la concentration en dioxyde de carbone dans un flux d'air produit par ledit échantillon d'haleine et dirigé à travers l'ensemble capteur ; et ladite unité centrale (21) peut être mise en oeuvre, essentiellement simultanément avec l'analyse desdites images, pour comparer ledit échantillon d'haleine à une valeur seuil (V) prédéfinie.

6. Dispositif selon la revendication 4, dans lequel ledit ensemble capteur comprend un capteur de dioxyde de carbone (18) agencé et configuré pour fournir un signal relatif au dioxyde de carbone (28) représentant la concentration en dioxyde de carbone dans un flux d'air produit par ledit échantillon d'haleine et dirigé à travers l'ensemble capteur ; ladite unité centrale (21) pouvant être mise en oeuvre pour comparer ledit signal relatif au dioxyde de carbone (28) à une valeur seuil (V) prédéfinie, et pour extraire lesdites images de la mémoire tampon (22) et exécuter ladite analyse sur les images extraites en réaction au fait que ledit signal relatif au dioxyde de carbone (28) dépasse ladite valeur seuil (V).

7. Dispositif selon la revendication 6, dans lequel ladite unité centrale (21) peut être mise en oeuvre pour déterminer un moment (T) auquel ledit signal relatif au dioxyde de carbone (28) dépasse ladite valeur seuil (V), et pour extraire de ladite mémoire tampon (22) et analyser une série desdites images correspondant à une période (t) prédéfinie, dont au moins le début précède ledit moment (T).

8. Procédé inviolable d'analyse d'un échantillon d'haleine, le procédé comprenant les étapes consistant à : mettre à disposition un dispositif analyseur d'haleine (1) comportant une zone d'entrée (2) et un ensemble capteur, la zone d'entrée (2) étant configurée pour recevoir un échantillon d'haleine d'un sujet testé par le biais d'un orifice d'entrée (6) et diriger l'échantillon vers l'ensemble capteur, l'ensemble capteur étant configuré pour fournir un signal représentant la concentration d'une substance volatile dans l'échantillon ; le procédé comportant en outre la mise à disposition d'un dispositif d'imagerie (9) orienté vers le visage (14) d'un sujet testé (10), la mise en oeuvre dudit dispositif d'imagerie (9) tandis que le sujet testé (10) fournit un échantillon d'haleine à travers ledit orifice d'entrée (6) de manière à capturer des images du visage (14) du sujet testé, et l'analyse desdites images pour produire un signal indiquant un échantillon d'haleine non falsifié en fonction de ladite analyse, le procédé étant **caractérisé en ce qu'**il comporte i) la reconnaissance d'une bouche (11) du sujet testé et la détermination si la bouche (11) est ouverte ou non en analysant la longueur d'un contour extérieur (30) de la bouche du sujet dans lesdites images ; et ii) la détection d'un objet de blocage (31) entre une bouche (11) du sujet testé et ledit dispositif analyseur d'haleine (1) en détectant une rupture dans ledit contour extérieur (30) de la bouche du sujet dans lesdites images, et la production d'un signal d'erreur en réaction à cela.

9. Procédé selon la revendication 8, comportant en outre l'analyse desdites images en comparant des images individuelles entre elles.

10. Procédé selon la revendication 8 ou la revendication 9, comportant en outre la reconnaissance de traits de visage (11, 12, 16) dans lesdites images.

11. Procédé selon l'une quelconque des revendications 8 à 10, comportant en outre l'étape consistant à enregistrer temporairement lesdites images du dispositif d'imagerie dans une mémoire tampon (22) avant ladite étape consistant à analyser les images.

12. Procédé selon l'une quelconque des revendications 8 à 11, dans lequel le procédé comporte, essentiellement simultanément à ladite étape consistant à analyser les images : l'utilisation d'un capteur de dioxyde de carbone (18) pour fournir un signal relatif au dioxyde de carbone (28) représentant la concentration en dioxyde de carbone dans ledit flux d'air, et la comparaison dudit signal relatif au dioxyde de carbone (28) à une valeur seuil (V) prédéfinie.

13. Procédé selon la revendication 11, le procédé comprenant en outre les étapes consistant à : utiliser un capteur de dioxyde de carbone (8) pour fournir un signal relatif au dioxyde de carbone (28) représentant la concentration en dioxyde de carbone dans ledit flux d'air ; comparer ledit signal relatif au dioxyde de carbone (28) à une valeur seuil (V) prédéfinie ; et, si ledit signal relatif au dioxyde de carbone (28) dépasse ladite valeur seuil prédéfinie, extraire lesdites images enregistrées de la mémoire tampon (22) et effectuer ladite analyse sur les images extraites.

14. Procédé selon la revendication 13, comprenant en outre les étapes consistant à : déterminer un moment (T) auquel ledit signal relatif au dioxyde de carbone (28) dépasse ladite valeur seuil (V) ; et extraire de ladite mémoire tampon (22) et analyser une série d'images correspondant à une période (t) prédéfinie, dont au moins le début précède ledit moment (T).
